# EUROPÄISCHE PATENTANMELDUNG

(11) **EP 4 015 007 A1**
(43) Veröffentlichungstag der Anmeldung: **22.06.2022**
(21) Anmeldenummer: 20020616.7
(22) Anmeldetag: 15.12.2020
(51) Int. Cl.: A61L 2/20

(54) **VERFAHREN ZUM ENTGASEN UND TROCKNEN VON GEGENSTÄNDEN NACH EINER ETHYLENOXID STERILISATION**

(71) Anmelder: Peter Osypka Stiftung Grenzach-Wyhlen, Stiftung des bürgerlichen Rechts, 79639 Grenzach-Wyhlen (DE)
(72) Erfinder: OSYPKA, Peter, 79639 Grenzach-Wyhlen (DE)

(57) **Zusammenfassung**

Die Erfindung betrifft ein Verfahren zur Sterilisation von bioresorbierbaren Produkten unter Verwendung von Ethylenoxid, welches die folgenden Schritte umfasst:
Verpacken eines bioresorbierbaren Produkts in einem Sterilisationsbeutel oder einer Sterilisationsfolie;
Einbringen des verpackten Sterilisationsguts (15) in die Kammer eines Sterilisators (1); Evakuieren der Kammer (1) mit der Vakuumpumpe (2) zur Luftentfernung auf 50-100 mbar; Einleiten von Wasserdampf (14) bei einer Temperatur von 35-60°C zur Konditionierung des Sterilisationsguts;
Einleiten von einem Gasgemisch bestehend aus 6-15% Ethylenoxid und Inertgas (13) bis zu einem Überdruck von bis zu 3.500 mbar abs. zur Sterilisation;
Verbleiben des Sterilisationsguts (15) in der Sterilisationskammer (1) in einem Zeitraum von 15 min bis 10 Stunden, vorzugsweise 1-4 Stunden, bei einer Temperatur von 35-60°C; Evakuieren des Ethylenoxid Gasgemisches über einen Katalysator (9) zur Umwandlung des Ethylenoxids in CO₂ und Wasser;
Starten der Desorption mit zyklischen Luftspülung- Vakuum Zyklen im Wechsel wobei der Evakuierungsdruck zwischen 30-200mbar liegt;
Nochmaliges Evakuieren mit der Prozess-Vakuumpumpe (2);
Füllen der Kammer mit Inertgas bis zu einem Druck von 100-500mbar abs.

Das Verfahren ist dadurch gekennzeichnet, dass nach dem Spülvorgang mit Inertgas die Prozess-Vakuumpumpe (2) deaktiviert und die Hochvakuumpumpe (3) angesteuert wird; wobei in der Sterilisationskammer(1) ein Vakuum von weniger als 4x10⁻³ mbar erzeugt wird; Fluten der Sterilisationskammer mit Stickstoff bis der atmosphärische Druck erreicht wird; Entnahme des sterilen bioresorbierbaren Produkts (16).

## Beschreibung

Die vorliegende Erfindung betrifft eine Vorrichtung und ein Verfahren zum Entgasen und Trocknen von Gegenständen, die einer EO-Sterilisation unterworfen worden sind.

Die Sterilisation von Gegenständen--vor allem von solchen Gegenständen die im medizinischen Gebiet Anwendung finden-- mit Hilfe von Ethylenoxid (EO) als Sterilisationsgas ist schon lange bekannt. So regelt beispielsweise die Norm DIN EN ISO 11135 die Sterilisation von Medizinprodukten mit Ethylenoxid. Ethylenoxid wirkt zuverlässig gegen Viren, Pilze und Bakterien.

Die EO-Sterilisation findet in einer verschlossenen Kammer statt und besteht aus den folgenden Schritten:
Luftentfernung, Konditionierung, Sterilisierung und der Entfernung des Ethylenoxids. Bei feuchtigkeitsempfindlichem Sterilisationsgut muss eine Trocknung nachgeschaltet werden. Dies geschieht nach den bisher durchgeführten Verfahren in einer gesonderten Trocknungskammer, das heißt das Sterilisationsgut muss der Sterilisationskammer entnommen werden und wird in die Trocknungskammer überführt.

Zur Konditionierung wird das Sterilisationsgut gemäß festgelegter Parameter vorerwärmt und befeuchtet. Zur Konditionierung wird Wasserdampf verwendet. Die Konditionierung wird bei Temperaturen (einstellbar) zwischen 35 und 60°C durchgeführt. Die Konditionierung kann durch einmaliges Fluten oder durch mehrmaliges Fluten der Sterilisationskammer mit dem Wasserdampf erfolgen. Das Befeuchten des Sterilisationsguts ist wichtig, da durch die Feuchtigkeit die Zelloberfläche der Viren, der Bakterien oder der Pilze aufquillt und das Ethylenoxid besser eindringen kann.

Nach der Konditionierung erfolgt die Sterilisation. Es gibt sowohl Unterdruck-, als auch Überdruckverfahren. Das bei DMB Apparatebau unter dem Namen SteriVIT (Marke 3020180136571) durchgeführte Verfahren arbeitet im Überdruck. Moderne Sterilisatoren arbeiten mit einem Gemisch aus 6-15% Ethylenoxid und Rest Kohlendioxid.

Die Sterilisation mit dem Überdruckverfahren erfolgt über einen Zeitraum von 15 Minuten bis zu mehreren Stunden bei Temperaturen von 35-60°C, vorzugsweise 45-55°C und Sterilisationsdrücken von 1.850 bis 3.500mbar abs.

Die Sterilisationszeit ist abhängig von dem Sterilisationsgut, der Art der Verpackung, der Sterilisationstemperatur, des Sterilisationsdrucks, der Wirkmittelkonzentration und der Geometrie der Produkte mit den entsprechenden Innenlumen. Die Wirkmittelkonzentration an Ethylenoxid kann variieren und liegt zwischen 200mg/L bis 930mg/L.

Nach der Sterilisation erfolgt die Desorption des Ethylenoxids. Dazu wird die Sterilisationskammer mit Hilfe einer Vakuumpumpe evakuiert und anschließend werden zyklisch Luftspülung bzw. Nachvakuum im Wechsel durchgeführt. Der Evakuierungsdruck liegt bei bekannten Verfahren zwischen 30-200mbar. Dieser Spül-Evakuierungsprozess wird mehrere Male wiederholt um sicherzustellen, dass die in der Norm EN ISO 10993-7 festgelegten Grenzwerte für EO und ECH (Ethylenchlorhydrin) eingehalten werden. Das Ethylenoxid wird durch eine nachgeschaltete Entsorgungseinrichtung katalytisch in CO2 und Wasser zerlegt.

Die Sterilisation von bioresorbierbaren Produkten, d.h. von Produkten die aus Materialien bestehen, die im Körper nach gewisser Zeit abgebaut werden oder sich auflösen wie Stents, Nahtmaterialien, chirurgische Netze, Fäden oder Folien oder Implantate, Befestigungselemente wie Platten, Schrauben oder Nägel - stellt eine besondere Herausforderung dar.

Neben der Entfernung von Ethylenoxid ist eine Trocknung notwendig. Diese Produkte müssen absolut trocken verpackt, gelagert und ausgeliefert werden, damit sie nicht schon vor dem Einbau oder dem Einbringen in den menschlichen Körper an Festigkeit verlieren.

Wie oben erwähnt findet die Trocknung in bekannten Verfahren in einem gesonderten Prozess statt.

Ausgehend hiervon ist die Aufgabe der vorliegenden Erfindung ein Verfahren und eine entsprechende Vorrichtung zu entwickeln mit der die Ethylenoxid-Sterilisation von bioresorbierbaren Produkten gegenüber bekannten Verfahren vereinfacht werden kann. Der Transport des Sterilisationsguts während der Sterilisation und der Nachbehandlung (entgasen und trocknen) soll entfallen. Ein ununterbrochener Arbeitsgang soll gewährleistet werden.

Die Aufgabe wird durch das Verfahren mit den Merkmalen von Patentanspruch 1 gelöst.

Die Erfindung betrifft somit ein Verfahren zur Sterilisation von bioresorbierbaren Produkten unter Verwendung von Ethylenoxid, welches die folgenden Schritte umfasst: Konditionierung, Sterilisation, Nachkonditionierung und die Trocknung in einer einzigen Kammer um Handhabungs- Fehler und/oder eine erneute Kontamination der Produkte mit Feuchte bzw. Bakterien zu unterbinden.

Die Schritte im Einzelnen:
Schritt 1:
   Verpacken eines bioresorbierbaren Produkts (Sterilisationsgut) in einem Sterilisationsbeutel oder einer Sterilisationsfolie (z.B. Alu, Papier, Tyvek, Tiefziehblister).
Schritt 2:
   Einbringen des verpackten bioresorbierbaren Produkts in die Kammer eines Sterilisators
Schritt 3:
   Konditionierung der Produkte
   Evakuieren der Kammer mit einer Prozess-Vakuumpumpe zur Luftentfernung auf 50-100 mbar und Einleiten von Wasserdampf bei einer Temperatur von 35-60°C (einstellbar) zur Konditionierung des Sterilisationsguts
Schritt 4:
   Sterilisation der Produkte
   Einleiten von einem Gasgemisch bestehend aus 6-15% Ethylenoxid und Inertgas bis zu einem Überdruck von bis zu 3.500 mbar abs. zur Sterilisation.
   Verbleiben des Sterilisationsguts in der Sterilisationskammer in einem Zeitraum von 15 min bis zu 10 Stunden, vorzugsweise 1-4 Stunden bei einer Temperatur von 35-60°C. Evakuieren des Ethylenoxid Gasgemisches über einen Katalysator zur Umwandlung des Ethylenoxids in CO₂ und Wasser
Schritt 5:
   Nachkonditionierung der Produkte
   Starten der Desorption mit zyklischen Luftspülung- Vakuum Zyklen im Wechsel. Die Evakuierung erfolgt mit der Prozess-Vakuumpumpe. Der Evakuierungsdruck liegt zwischen 30-200mbar. Dieser Spül-Evakuierungsprozess wird mehrere Male wiederholt, beispielsweise 10-40 Mal. Die zyklischen Druckwechsel in der Desorption reduzieren den Feuchtigkeitsgehalt in den Produkten auf ca. 10-20% rel. Feuchte.
Schritt 6:
   Trocknung
   Einleiten der Trocknung. Die Prozess-Vakuumpumpe evakuiert nochmals den Kammerinhalt und füllt anschließend die Kammer mit Inertgas (Stickstoff) bis zu einem Druck von 100-500mbar abs., dadurch wird nochmals ein Medium hinzugegeben, das nochmals vorhandene Feuchte aus dem Produkt aufnehmen kann. Der Kammermantel der Sterilisationskammer wird dabei weiter beheizt (einstellbar 35 - 60°C). Das Verfahren ist dadurch gekennzeichnet, dass nach dem Spülvorgang mit Inertgas (Stickstoff) die Prozess-Vakuumpumpe deaktiviert und die Hochvakuumpumpe angesteuert wird. Um den Pumpenwechsel vornehmen zu können, ist die Sterilisationskammer entweder mit einem Anschluss Stutzen der 2 Ventile trägt ausgestattet oder mit zwei Anschluss-Stutzen mit jeweils einem Ventil. Die Hochvakuumpumpe erzeugt in der Sterilisationskammer ein Vakuum von weniger als 4x10⁻³ mbar.

Durch Erzeugen dieses Vakuums wird die noch vorhandene Feuchtigkeit aus dem bioresorbierbaren Produkt weiter verdampft und die Restfeuchtigkeit der Produkte fällt auf ein Minimum (wenige ppm). Des Weiteren, kann durch das tiefe Vakuum noch geringe Bestandteile von Ethylenoxid aus dem Produkt gelöst und entsorgt werden. Wenn die Trocknung gestoppt wird, wird die Sterilisationskammer mit Stickstoff geflutet bis Atmosphärendruck erreicht ist, um Re-Kontamination mit Feuchtigkeit zu unterbinden.

Das Anschließen einer Hochvakuumpumpe an die Sterilisationskammer ist keineswegs trivial. So besteht die Schwierigkeit in:

### Sterilisationskammer

Die Sterilisationskammer muss für ein Vakuum von 4x10⁻³ mbar hergestellt werden.
Für den Pumpenstand (Trocknung) wird ein geeigneter Anschluss an die Sterilisationskammer benötigt (min. 2 Zoll)

### Kammerdichtung:

Als Abdichtung der Sterilisationskammer dienen zwei O-Ring Dichtungen, die druckbeaufschlagt werden. Durch die Beaufschlagung mit Druckluft werden die Dichtungen aus der Dichtungsnut gegen die Kammertüren gedrückt. Hiermit wird gewährleistet, dass die Kammertüren hermetisch abgedichtet sind und im Hochvakuum keine Leckage entstehen kann.

### Kammergröße

Die Trocknungspumpe muss entsprechend der Kammergröße ausgelegt werden. Die Sterilisationskammern bestehen komplett aus Edelstahl (Innenmantel und Heizungsmantel). Die aktuelle maximale Größe ist eine 3.290 Liter Sterilisationskammer mit entsprechender Trocknungspumpe. Die meisten Kunden mit bioresorbierbarem Material verwenden Sterilisationskammern mit einem Inhalt von 500 bis 1.050 Liter!

### Prozess-Vakuumpumpe und Trocknungspumpe

Bei Sterilisationskammern bis 1.050 Liter wird die Prozess-Vakuumpumpe eine Wasserringpumpe sein und bei Sterilisationskammern bis 3.290 Liter eine trocken verdichtende Schraubenpumpe.

Die Hochvakuumpumpe (Pumpenstand) kann entweder eine Kombination aus Wälzkolbenpumpe und Drehschieberpumpe sein (für Sterilisatoren bis 1.050 Liter) oder aus einer Kombination aus trocken verdichtender Schraubenpumpe und Wälzkolbenpumpe (für Sterilisatoren bis 3.290 Liter) die das o.g. Vakuum von 4x10⁻³ mbar erreichen. Der Pumpenstand wird nicht mit hohen Wirkmittel-Konzentrationen in Verbindung kommen, da diese schon über die Prozess-Vakuumpumpe evakuiert wurden. Dadurch muss der Pumpenstand auch keinen ATEX-Schutz besitzen noch Abdichtungen der Pumpenwellen aufweisen die beständig gegen hohe Wirkmittel-Konzentrationen sind.

### Allgemeine Ventile die an die Sterilisationskammer angeschlossen sind

Alle Ventile die an der Sterilisationskammer angeschlossen sind, müssen dem Vakuum von 4x10⁻³ mbar standhalten und wiederum einem Sterilisationsdruck von max. 3.500 mbar abs. beständig sein

Um eine Freigabe der Trocknung durchzuführen wird ein Drucksensor benötigt, der das Vakuum von 4x10⁻³ mbar anzeigen kann und für einen Sterilisationsdruck von max. 3.500 mbar abs. beständig ist.

### Ziel der Erfindung:

Durch die integrierte Trocknung können die Prozesszeiten signifikant reduziert werden. In den derzeit eingesetzten Trocknungsschränken benötigt man für die Trocknung bis zu 72 Stunden. Hinzu kommen die Zeiten für das Handling der Produkte (Zusätzlicher Be- und Entladevorgang) und der gesamte Sterilisationszyklus selbst. Somit dauert ein kompletter Zyklus bis bioresorbierbare Produkte verwendungsfähig vorliegen bis ca. 84h.

Durch die oben beschriebene Technologie werden wir den gesamten Zyklus für bioresorbierbare Produkte auf ca. 15h reduzieren. Das ist eine Reduktion der Zeit von ca. 82% zu der konventionellen Technologie. On top werden Handlings Fehler und Rekontaminationen ausgeschlossen.

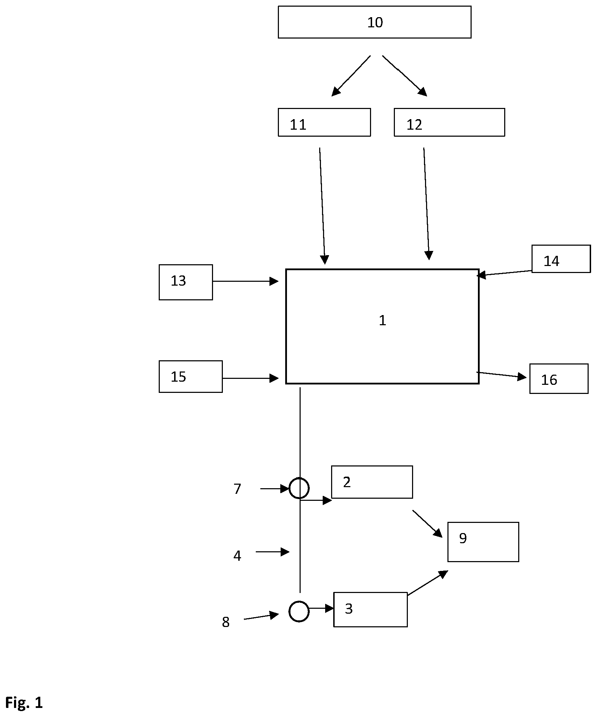

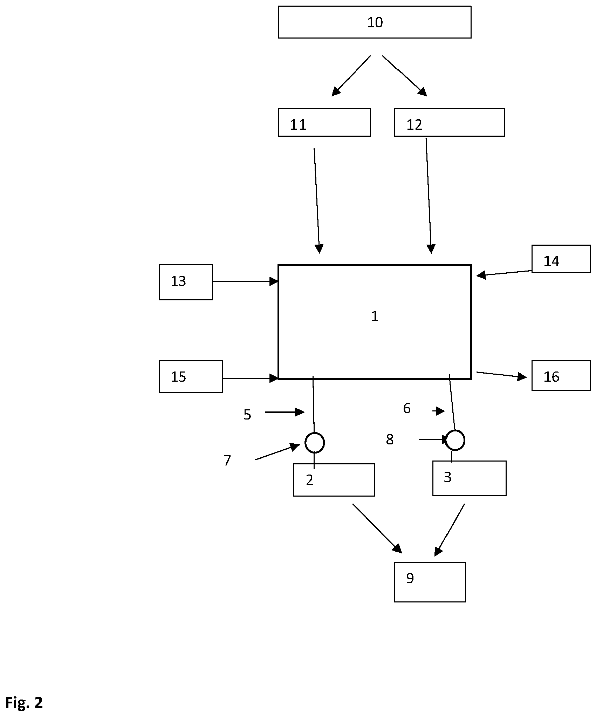

Die Vorrichtung ist skizzenhaft in Figur 1 und 2 dargestellt. Die Figuren zeigen zwei Varianten der erfindunsgemässen Vorrichtung.

Fig. 1 zeigt die Variante mit einem Anschluss-Stutzen an der Sterilisationskammer. In Fig. 2 hat die Sterilisationskammer 2 Anschlussstutzen.

### Die Bezugszeichen bedeuten

- 1: Sterilisationskammer
- 2: Prozess-Vakuumpumpe
- 3: Hochvakuumpumpe
- 4: Anschluss-Stutzen für 2 Pumpen
- 5: Anschluss-Stutzen für die Prozess-Vakuumpumpe
- 6: Anschluss-Stutzen für die Hoch-Vakuumpumpe
- 7: Ventil zur Prozess-Vakuumpumpe
- 8: Ventil zur Hochvakuumpumpe
- 9: ETO Entsorgungseinrichtung/Katalysator.
- 10: Steuerungseinrichtung zur Steuerung von Zeit (11) und Druck (12)
- 13: ETO/Stickstoff-Zufuhr
- 14: Wasserdampfzufuhr
- 15: unsteriles Produkt
- 16: steriles Produkt

Fig. 1 zeigt schematisch die abgedichtete implosionssichere Sterilisationskammer (1) mit einem einzigen Anschluss-Stutzen (4) für zwei Vakuumpumpen, die Prozess-Vakuumpumpe (2) und die Hochvakuumpumpe (3). Der Stutzen (4) hat 2 Ventile; Ventil (7) für die Prozess-Vakuumpumpe (2) und Ventil (8) für die Hochvakuumpumpe (3). Ein Steuerungsmechanismus (10) regelt die nötigen Prozessparameter wie beispielsweise die Sterilisationszeit (11) und den Druck (12). Das unsterile verpackte Produkt (15) wird vor Prozessbeginn in die Sterilisationskammer (1) eingebracht und verbleibt dort während des gesamten Prozesses. Nach Prozessende einschließlich Trocknung verlässt das nun sterile Produkt (16) die Kammer. Eine Wasserdampfzufuhr (14) und ETO/Stickstoff-Zufuhr (13) führt in die Kammer. Nach der Sterilisation wird das ETO über die ETO Entsorgungseinrichtung (9) katalytisch zersetzt.

Fig. 2 zeigt im Unterschied zu Fig. 1 die Sterilisationskammer (1) mit zwei Anschluss-Stutzen (5), (6). Anschluss-Stutzen (5) führt über Ventil (7) zur Prozess-Vakuumpumpe (2) und Anschluss-Stutzen (6) führt über Ventil (8) zur Hochvakuumpumpe (3). Die restlichen Bezugszeichen entsprechen den Bezugszeichen in Fig. 1.

## Patentansprüche

1. Verfahren zur Sterilisation von bioresorbierbaren Produkten unter Verwendung von Ethylenoxid, welches die folgenden Schritte umfasst:
Verpacken eines bioresorbierbaren Produkts in einem Sterilisationsbeutel oder einer Sterilisationsfolie;
Einbringen des verpackten Sterilisationsguts (15) in die Kammer eines Sterilisators (1);
Evakuieren der Kammer (1) mit der Vakuumpumpe (2) zur Luftentfernung auf 50-100 mbar;
Einleiten von Wasserdampf (14) bei einer Temperatur von 35-60°C zur Konditionierung des Sterilisationsguts;
Einleiten von einem Gasgemisch bestehend aus 6-15% Ethylenoxid und Inertgas (13) bis zu einem Überdruck von bis zu 3.500 mbar abs. zur Sterilisation;
Verbleiben des Sterilisationsguts (15) in der Sterilisationskammer (1) in einem Zeitraum von 15 min bis 10 Stunden, vorzugsweise 1-4 Stunden, bei einer Temperatur von 35-60°C;
Evakuieren des Ethylenoxid Gasgemisches über einen Katalysator (9) zur Umwandlung des Ethylenoxids in CO₂ und Wasser;
Starten der Desorption mit zyklischen Luftspülung- Vakuum Zyklen im Wechsel wobei der Evakuierungsdruck zwischen 30-200mbar liegt;
Nochmaliges Evakuieren mit der Prozess-Vakuumpumpe (2);
Füllen der Kammer mit Inertgas bis zu einem Druck von 100-500mbar abs,
**dadurch gekennzeichnet, dass** nach dem Spülvorgang mit Inertgas die Prozess-Vakuumpumpe (2) deaktiviert und die Hochvakuumpumpe (3) angesteuert wird; wobei in der Sterilisationskammer(1) ein Vakuum von weniger als 4x10⁻³ mbar erzeugt wird;
Fluten der Sterilisationskammer mit Stickstoff bis der atmosphärische Druck erreicht wird;
Entnahme des sterilen bioresorbierbaren Produkts (16).

2. Verfahren nach Anspruch 1, **dadurch gekennzeichnet, dass** nach dem Spülvorgang mit Inertgas die Prozess-Vakuumpumpe (2) deaktiviert und die Hochvakuumpumpe (3) angesteuert wird, wobei die Steuerung der Prozess-Vakuumpumpe (2) und der Hochvakuumpumpe (3) über einen einzigen Anschluss-Stutzen (4) erfolgt.

3. Verfahren nach Anspruch 1, **dadurch gekennzeichnet, dass** nach dem Spülvorgang mit Inertgas die Prozess-Vakuumpumpe (2) deaktiviert und die Hochvakuumpumpe (3) angesteuert wird, wobei die Steuerung der Prozess-Vakuumpumpe (2) und der Hochvakuumpumpe (3) über zwei Anschluss-Stutzen (4) und (5) erfolgt.
